# EUROPEAN PATENT SPECIFICATION

(11) **EP 1 448 184 B1**
(45) Date of publication and mention of the grant of the patent: **19.04.2006**
(21) Application number: 02793337.3
(22) Date of filing: 26.11.2002
(51) Int. Cl.: A61K 31/205, A61K 31/277, A61P 15/00

(54) **USE OF PROPIONYL L-CARNITINE OR ONE OF ITS PHARMACOLOGICALLY ACCEPTABLE SALTS FOR THE PREPARATION OF A MEDICINE FOR THE TREATMENT OF LA PEYRONIE S DISEASE**
VERWENDUNG VON PROPIONYL-L-CARNITIN ODER DESSEN PHARMAKOLOGISCH AKZEPTABLER SALZE ZUR HERSTELLUNG EINES MEDIKAMENTES ZUR BEHANDLUNG VON LA PEYRONIE'S KRANKHEIT
UTILISATION DE PROPIONYL L-CARNITINE OU D'UN DE SES SELS PHARMACEUTIQUEMENT ACCEPTABLES DANS LA FABRICATION D'UN MEDICAMENT DESTINE AU TRAITEMENT DE LA MALADIE DE LA PEYRONIE

(30) Priority: 26.11.2001 IT RM20010695
(43) Date of publication of application: 25.08.2004
(73) Proprietor: SIGMA-TAU Industrie Farmaceutiche Riunite S.p.A., 00144 Roma (IT)
(72) Inventor: Koverech, Aleardo, Sigma-Tau Ind.Farm.Riunite SPA, I-00040 Pomezia (IT); CAVALLINI, Giorgio, I-44100 Ferrara (IT); BIAGIOTTI, Giulio, I-06070 Perugia (IT)
(74) Representative: Spadaro, Marco
(86) International application number: PCT/IT2002/000745
(87) International publication number: WO 2003/045373

(56) References cited:
- EP-A- 0 797 993
- BIAGIOTTI G ET AL: "ACETYL-L-CARNITINE VS TAMOXIFEN IN THE ORAL THERAPY OF PEYRONIE'S DISEASE: A PRELIMINARY REPORT" BJU INTERNATIONAL, BLACKWELL SCIENCE, OXFORD, GB, vol. 88, no. 1, July 2001 (2001-07), pages 63-67, XP001106370 ISSN: 1464-4096 cited in the application
- CAVALLINI G ET AL: "Oral propionyl-L-carnitine and intraplaque verapamil in the therapy of advanced and resistant Peyronie's disease." BJU INTERNATIONAL, vol. 89, no. 9, June 2002 (2002-06), pages 895-900, XP001146228 June, 2002 ISSN: 1464-4096

## Description

The invention described herein relates to the use of propionyl L-carnitine or one of its pharmacologically acceptable salts for the preparation of a medicine useful in the treatment of La Peyronie's disease.

### Background to the invention

La Peyronie's disease is an inflammatory condition still of largely unclear aetiology (probably hereditary-based autoimmune) affecting the tunica albuginea of the penis. This inflammation is self-maintaining and is prevalently sectorial (*Belgrano E. et al. ed. Induratio Penis Plastica: Stato Dell'Arte. Ospedaletto (Pisa); Pacini Editore, 1999*). Only in some cases, in fact, does it affect other sheaths of the body (palmar aponeurosis, retroperitoneal fascia). One can distinguish between three stages of La Peyronie's disease, namely, acute, early chronic and late chronic (advanced), each with its own histological, symptomatological and physical and instrumental semeiological characteristics. The stages are determined on the basis of symptoms, physical examination, colour Doppler ultrasonography and histopathological models. Though the models of the disease are consistent, the same cannot be said of its duration, extent and severity (*Davis C, J. Urol. 1997; 157; 272-5*). Currently, there exist a whole series of pharmacological and physical remedies in the therapy of La Peyronie's disease, which is probably the disease associated with the largest number of non-surgical attempts at treatment, owing to the paucity of case-control studies and the peculiarity that the disease progresses in some 10-20% of cases despite the therapy (forms indicated herein as "resistant La Peyronie's disease").

The therapy of the disease is now well standardised from the point of view of the timing of therapy: the first approach in order of time is pharmacological, and then later, whenever the progression of the disease has been arrested and medical therapy has proved insufficient to remedy the deformity of the penis or the erectile deficit induced by La Peyronie's disease, surgical therapy is resorted to. The first approach (medical) consists in the administration of drugs via several routes and is mainly indicated in the acute and early chronic stages (*Belgrano, ibid*). Various drugs have been tried, such as tocopherols, vitamin E, para-aminobenzoic acid, allopurinol, colchicines, immunomodulators and tamoxifen. Apart from the tocopherols, vitamin E and allopurinol, the other drugs have shown a certain measure of efficacy, which is also predictable on a scientific basis, but present the occurrence of unwanted side effects such as gastrointestinal symptoms, reduced libido, skin rashes, and fever in 18-33% of patients, thus limiting or contraindicating their use (*Belgrano, ibid.*).

The occurrence of side effects and sometimes difficulties with the manageability of certain drugs (immunomodulators, tamoxifen) have posed the problem of the need to look for alternative drugs for the treatment of La Peyronie's disease. This search is aggravated by the fact that there are currently no reliable experimental models of La Peyronie's disease. Effectively speaking, reports have recently been published on the induction of La Peyronie's-disease-like lesions in rats by the injection of the cyctokine, transforming growth factor beta 1, into the tunica albuginea. This substance transforms fibrocytes into fibroblasts and increases vascularisation and vascular permeability by inducing angiogenesis. (*El-Sakka A.I., et al. Br. J. Urol. 1998, 81:445-52; Bivalacqua T.J., et al. J. Androl. 2001, 22:497-506; Bivalacqua T.J., et al. J. Urol. 2000, 163:1992-8; El-Sakka A.I., J. Urol. 1997, 158:2284-90*). This model is based on the assumption, reiterated several times by the authors, that La Peyronie's disease is due to an exaggerated cicatricial response following a genital trauma. This view must be regarded as superseded by the hypothesis that the disease is based on a hereditary autoimmunity of two types, one consisting in the total penetrance of an autosomal dominant gene probably related to the HLA (histocompatibility) locus of chromosome 6 (rare form - 1 case out of 4 - which is inherited from father to son) and the other in partial penetrance (more frequent) in which a predisposition to fibrosclerosing autoimmune diseases is inherited (*Belgrano E., ibid.; Aynaud O., Casanova J.M.: Pathologie de la Verge. Masson, Parigi 1998*). Moreover, the experimental model is induced, whereas human La Peyronie's disease arises spontaneously. This model is based on injection into the tunica albuginea of transforming growth factor beta 1, which is a cytokine produced by the endothelium both in the inflammatory phase (*Miculeck A.A., et al. Arch. Facial Plast. Surg. 2001: 3: 111-114*) and in the course of an increased metabolic requirement (*Fasciani A., et al. Fertil. Steril. 75: 1218-1221, 2001*), and is therefore an entirely aspecific cytokine (and thus experimental model). What is more, in La Peyronie's disease, as in all other forms of inflammation of the body, a large quantity of cytokines are secreted, which, when generically injected into a mammal, cause inflammation in a completely aspecific manner (*Belgrano E., ibid.; Aynaud O., Casanova J.M., ibid.*).

The experimental model implies that the erectile deficit secondary to La Peyronie's disease is due to an arterial deficiency. Recent research, however, has shown that the erectile deficit secondary to La Peyronie's disease is secondary to a venous deficiency (*Belgrano E., ibid.; Aynaud O., Casanova J.M. ibid.*).

Furthermore, La Peyronie's disease, being a form of inflammation of likely autoimmune origin, is self-maintaining and mainly sectorial, as mentioned above, whereas this is not the case in the experimental model.

At the present time there are no reliable experimental models of La Peyronie's disease, since it constitutes a form of inflammation of as yet unknown pathogenesis.

Two case-control studies using drugs are currently known to have been conducted, one comparing acetyl-carnitine versus tamoxifen (*Biagiotti G., Cavallini G.: BJU International (201), 88, 63-67*) and the other tamoxifen versus placebo (*Teloken C., et al. J. Urol. 162 2003-2005, 1999*) in a patient population not selected on the basis of stage.

No case-control studies are known to exist on the advanced stages of the disease; moreover, in the literature there are no reports on Petronie's disease resistant to medical therapy, which constitutes a class destined to remain unhealed, where even surgery is not a feasible proposition (reserved exclusively for those subjects in whom progression of the disease has been arrested by medical therapy) and no wholly reliable pharmacological therapy is yet available.

Medical therapies for La Peyronie's disease sometimes entail modes of administration which are distinctly unpleasant for the patient: subcutaneous injections adjacent to the plaque (*Brake M. et al.; BJU Int. 2001, May; 87(7):654-657*, where, amongst other things, the unsatisfactory outcome of treatment with interferon 2β is reported); local injections of betamethasone, hyaluronidase and lidocaine; or intralesional injections of verapamil (*Lamprakopulos A. et al. Scand. J. Urol. Nephrol. 2000 Dec.; 34(6):355-360; Rehman et al. Urology, 1998, Apr,; 51 (4); 620-626*).

In addition to the extensive scientific literature, the patent literature reports various different methods of treating the disease with different active ingredients, see, for example, WO 01/17479, Androsolutions, which involves the administration of drugs via an intrapenile catheter; WO 01/09178, Incyte Genomics, that utilises chaperonine; the various Vivus Inc patents (US 6,113,393; US 5,925,629, US 5,773,020, US 5,474,535 and EP 0 526 566), and many others, in which the most commonly indicated administration modes are of the intraurethral type. Other patents worthy of mention are US 6,093,181, BR 9801985 (one of the few to provide a topical treatment with a cream), US 6,033,374, US 6,031,005, EP 1 097 202, US 6,022,539, EP 0 986 417, and US 4,338,300.

It is therefore necessary to have a therapy for La Peyronie's disease that matches up to the requirements of efficacy with an administration mode which is well accepted by the patient, e.g. by mouth, and that is also capable of resolving cases resistant to the known treatments.

The aim of the invention described herein is to meet these requirements and solve the above-mentioned problems.

Propionyl L-carnitine is known to have numerous therapeutic uses: for example, US-A-4 415 589 describes its use, together with other lower acyl carnitines, for the treatment of diseases of the veins, such as venous stasis, the aetiology of which is based on the reduced elasticity of the erythrocyte wall. European Patent EP 0 793 962 describes the use of propionyl L-carnitine in the treatment of Stage 2 chronic obliterating atherosclerosis according to the Leriche Fontaine classification (intermittent claudication), a disease of the lower limbs caused by an inadequate blood supply to the muscles. US Patent 5,869,528 describes the use of L-carnitine and the lower acyl L-carnitines, including propionyl, in the treatment of attention deficit and hyperactivity disorder (ADHD). US Patent 6,013,670 describes the use of propionyl L-carnitine in the intrarectal treatment of chronic inflammation of the bowel. All the above-mentioned patents are filed in the name of the Applicant.

A propionyl L-carnitine-based drug is commercially available in Italy under the trade name Dromos® , indicated for the treatment of obliterating arteriopathy of the lower limbs and for the therapy of chronic congestive heart failure in order to increase the tolerance of physical effort (see also patent GB 2,008,578). EP-A-0 797 993 discloses pharmaceutical composition comprising propionyl-L-carnitine and vitamin E.

No efficacy of propionyl L-carnitine has ever been described in the treatment of diseases such as La Peyronie's disease characterised by fibrotic tissue.

In the above-cited article by Biagiotti and Cavallini (*BJU International 88: 63-67, 2001*) it was demonstrated that acetyl L-carnitine is significantly more active than tamoxifen in the treatment of La Peyronie's disease in the early stages. In this study the authors limited the use of acetyl L-carnitine only to the oral treatment of the acute and early chronic stages, stating that the more severe chronic and resistant states were intractable with oral therapy alone. Since the aetiology of La Peyronie's disease has yet to be fully clarified (*Hellstrom WJ and Bivalacqua TJ, J. Androl. 2000 May-Jun; 21(3):347-54*)*,* it is putatively suggested that, on the basis of several of its known properties, acetyl L-carnitine may conceivably play a role in the inflammation and fibrosis typical of the disease without, however, in any way suggesting the use of propionyl L-carnitine, which, in any event, is a different molecule.

Propionyl L-carnitine has proved capable of affording protection in experimental animal models (rats) against forms of chemically induced inflammation of both the skin and the arteries (*Amico-Roxas M. et al. Drug Exptl. Clin. Res. 19: 213-217,* 1993; *Corsico N. et al., Cardiovasc. Drugs Ther. 7: 341-351, 1993*), this property not being shared by acetyl L-carnitine and L-carnitine. It should be noted that the animal model used by Amico Roxas *et al*. Is not in any way transferable to or even predictive for a fibrosis-based disease such as La Peyronie's. The Roxas model, in actual fact, is a model of cutaneous inflammation induced by chemical agents, whereas in the case of La Peyronie's disease an autoimmune pathogenesis is postulated (*Belgrano e al. ibid.*).

Various considerations based on the existing state of knowledge of the carnitine system substantiate the claim that this difference in activity between acetyl L-carnitine and propionyl L-carnitine in Amico Roxas *et al.*'s inflammation model is not attributable to a greater bio-availability of the different acyl esters at tissue level, since the amount bound and that present in solution in plasma are the same and cannot be modified by external inputs (*Marzo A. et al., Eur. J. Drug Metab. Pharmacokinet. 3:364-368, 1991*), but only by actual biochemical differences between the respective esters.

### Summary of the invention

It has now been found that propionyl L-canitine is effective in the treatment of La Peyronie's disease to an even greater extent than acetyl L-carnitine.

Accordingly, one object of the invention described herein is the use of propionyl L-carnitine or one of its pharmaceutically acceptable salts for the preparation of a medicine useful for the treatment of La Peyronie's disease, in its various different forms and stages, particularly in the acute, early chronic and late chronic stages and in the resistant forms. In particular, said medicine is suitable for oral, intravenous, intramuscular and intraplaque administration.

Another object of the invention described herein is a combination of propionyl L-carnitine, or an equivalent amount of one of its pharmaceutically acceptable salts, and one or more ingredients active in the therapy of La Peyronie's disease. Though no restrictions apply with regard to the type of active ingredient to be used in the therapy of La Peyronie's disease, the following substances are indicated by way of examples: tocopherols, vitamin E, allopurinol, potassium aminobenzoate (POTABA), tamoxifen, immunomodulators, triamcinolone, and verapamil. The expert in the sector is capable of determining the active ingredient in the therapy of La Peyronie's disease on the basis of his or her general knowledge, such as, for example, the above-cited report by Belgrano and the literature cited in this patent application.

In one particular aspect of the invention, the combination consists of propionyl L-carnitine and verapamil.

Said combination can be formulated in a pharmaceutical composition, which is also an object of the invention described herein, useful as a medicine for the treatment of La Peyronie's disease.

Propionyl L-carnitine lends itself to the treatment of La Peyronie's disease in its broadest definition, but has proved particularly useful in patients with La Peyronie's disease at the chronic advanced stage and, surprisingly, in patients with La Peyronie's disease resistant to other "conventional" forms of therapy.

### Detailed description of the invention

According to the invention described herein, propionyl L-carnitine can be used both in the form of an inner salt, which is in itself pharmaceutically acceptable, and in the form of a salt with a pharmaceutically acceptable acid.

What is meant by pharmaceutically acceptable salt of propionyl L-carnitine is any of its salts with an acid that does not give rise to unwanted toxic or side effects.

Such acids are well known to pharmacologists and to experts in pharmaceutical technology.

Examples of such salts, though by no means constituting a complete list, are: chloride, bromide, orotate, acid aspartate, acid citrate, magnesium citrate, acid phosphate, fumarate and acid fumarate, magnesium fumarate, lactate, maleate and acid maleate, mucate, acid oxalate, pamoate, acid pamoate, acid sulphate, glucose phosphate, tartrate, acid tartrate, magnesium tartrate, 2-amino-ethane-sulphonate, magnesium 2-amino-ethane-sulphonate, 2-amino-ethane-sulphonate chloride, glycine chloride, choline tartrate, taurinate and trichloroacetate.

In an initial realisation form of the invention described herein, the medicine containing propionyl L-carnitine is suitable for oral administration.

In the case of the combination of propionyl L-carnitine and another active ingredient, as seen above, particularly verapamil, such a combination lends itself to co-ordinated use.

Within the context of the invention "for co-ordinated use" of the aforementioned compounds what is meant, indifferently, is either any form of co-administration, i.e. the essentially simultaneous or sequential administration of propionyl L-carnitine or one of its pharmacologically acceptable salts and the other active ingredient, or the administration of a composition containing the aforesaid active ingredients in a combination and mixture, in addition to excipients, if any.

In this context, the medicine, or the pharmaceutical composition described here below, containing the combination, shall be formulated in such a way as to allow the administration of the two active ingredients in the different modes envisaged for co-ordinated use. In a preferred realisation of this aspect of the invention described herein, said medicine is suitable for the oral administration of propionyl L-carnitine and for the intraplaque administration of verapamil.

The medicine, particularly for co-ordinated use, shall be conveniently prepared in the form of a pharmaceutical composition. According to the invention described herein, the active ingredient shall be in a mixture with suitable vehicles and/or excipients commonly used in pharmacy, such as, for example, those described in "Remington's Pharmaceutical Sciences Handbook", latest edition. The compositions according to the invention shall contain a therapeutically efficacious amount of the active ingredient. The dosages and modes of administration, even when envisaged for co-ordinated use, shall be determined by the expert in the sector, e.g. the clinician or primary care physician, according to the type of disease to be treated and the patient's condition, or, concomitantly with the administration of other active ingredients.

Examples of pharmaceutical compositions are those that allow oral or parenteral administration by the intravenous, intramuscular, subcutaneous, or transdermal routes. Suitable pharmaceutical compositions for the purpose are tablets, rigid or soft capsules, powders, solutions, suspensions, syrups, and solid forms for extempore liquid preparations. Compositions for parenteral administration are, for example, all the intramuscular, intravenous and subcutaneous forms, in the form of solutions, suspensions and emulsions. Liposomal formulations should also be mentioned. Also included are the forms involving the controlled release of the active ingredient, both for oral administration, including tablets coated with appropriate layers, microencapsulated powders, complexes with cyclodextrin, and depot forms, e.g. subcutaneous ones, such as depot injections or implants. The preferred administration forms for propionyl L-carnitine are the oral, intramuscular, intravenous and intraplaque forms.

In a preferred realisation form of the invention described herein, the medicine, particularly for co-ordinated use, is suitable for the oral administration of 1 g of propionyl L-carnitine, or an equivalent amount of one of its pharmaceutically acceptable salts, per dosage unit, and for the intraplaque administration of 10 mg of verapamil.

What is meant by preferred form is the mode of administration of propionyl L-carnitine preferred by the patients, which has been used extensively in its oral formulation, but which has proved efficacious in the therapy of La Peyronie's disease at the chronic advanced stage also in its intramuscular, intravenous and intraplaque form. These alternative administration routes (intramuscular or intravenous) have been used in cases in which the patients refused oral administration or presented unavoidable side effects (hypotension) as a result of the intraplaque injection of verapamil.

The following examples further illustrate the invention.

The examples are based on a limited number of patients and therefore the analysis of the results was carried out only with regard to the fundamental characteristics of the disease, namely plaque area in mm² (as measured by means of dynamic colour Doppler ultrasonography) and curvature of the artificially erect penis induced by intracavernous injection of 20 µg of prostaglandin El.

### Example 1

### Oral tamoxifen versus intravenous propionyl L-carnitine

40 subjects (mean age 52, range 44-61) with induratio penis plastica (La Peyronie's disease) at the chronic stage were randomised to two groups of 20 patients each. The first group was submitted to therapy with intravenous propionyl L-carnitine 300 x 2 mg/day for 3 months, and the second group to therapy with oral tamoxifen 20 x 2 mg/day. The variables measured were plaque area by means of dynamic colour Doppler ultrasonography and penile curvature, which was present in 16 patients per group. It was found that intravenous propionyl L-carnitine reduces the plaque area and the curvature of the artificially erect penis to a significantly greater extent than tamoxifen. Intraplaque verapamil was not used in these patients.

The results are presented in Table 1, where the data are expressed as mean ± standard deviation (S.D.).

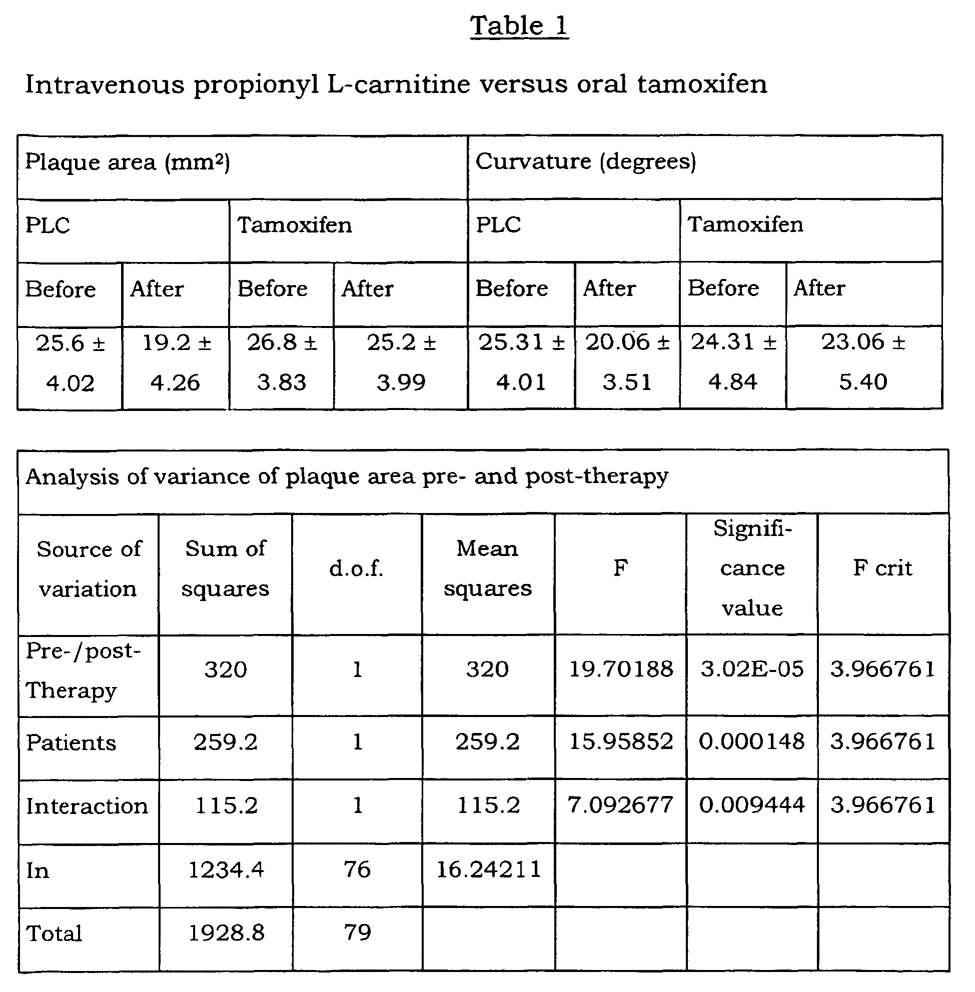

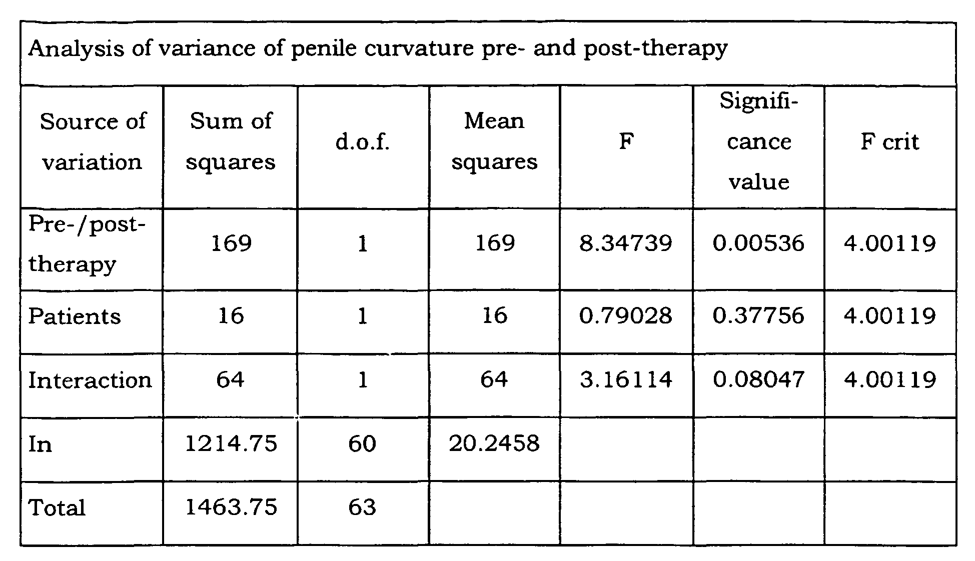

### Example 2

### Oral tamoxifen versus intramuscular propionyl L-carnitine

30 subjects (mean age 50, range 42-63) with induratio penis plastica in the early chronic stage were randomised to two groups of 15 patients each. The first group was submitted to therapy with intramuscular propionyl L-carnitine 300 x 2 mg/day for 3 months, and the second group to therapy with oral tamoxifen 20 x 2 mg/day. The variables measured were plaque area (in all subjects) and penile curvature, which was present in 12 subjects per group. It was found that intramuscular propionyl L-carnitine reduces the plaque area and the curvature of the artificially erect penis to a significantly greater extent than tamoxifen. Intraplaque verapamil was not used in these patients.

The results are presented in Table 2, where the data are expressed as mean ± standard deviation (S.D.).

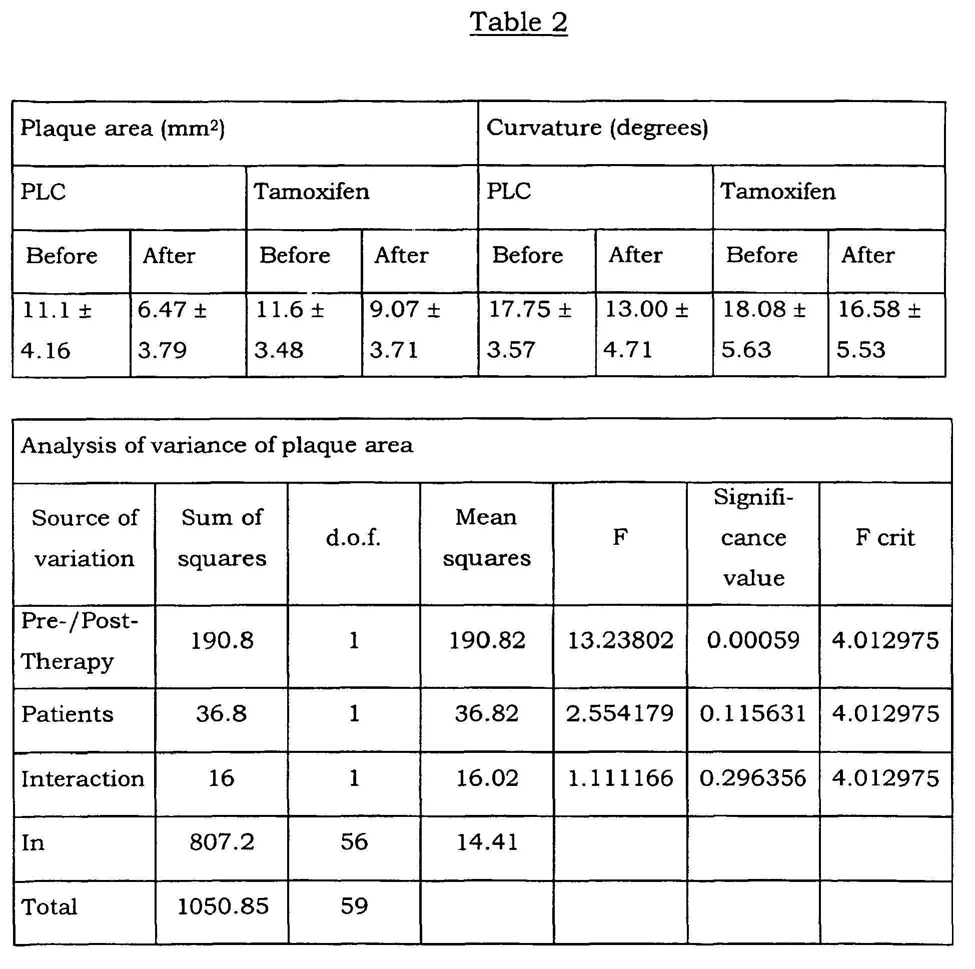

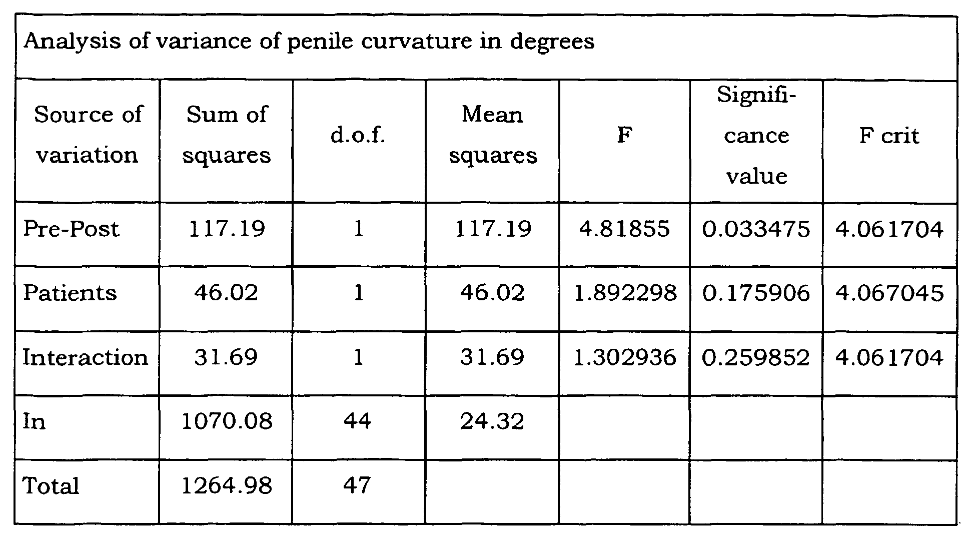

### Example 3

### Intraplaque triamcinolone acetonide versus intraplaque propionyl L-carnitine.

In this case 34 the study population consisted of 34 patients (mean age 50, range 44-63) with induratio penis plastica in the chronic stage, who presented an irremediable intolerance to intraplaque verapamil.

17 were submitted to intraplaque infiltrations with propionyl L-carnitine 300 mg, 1 infiltration per week for 10 weeks, and 17 to intraplaque infiltrations with triamcinolone acetonide 40 mg (Kenacort® Squibb), 1 infiltration per week for 10 weeks. The variables measured were plaque area in mm² by dynamic colour Doppler ultrasonography and the curvature of the artificially erect penis in degrees.

Intraplaque propionyl L-carnitine proved significantly more active than triamcinolone acetonide.

The results are presented in Table 3, where the data are expressed as mean ± standard deviation (S.D.).

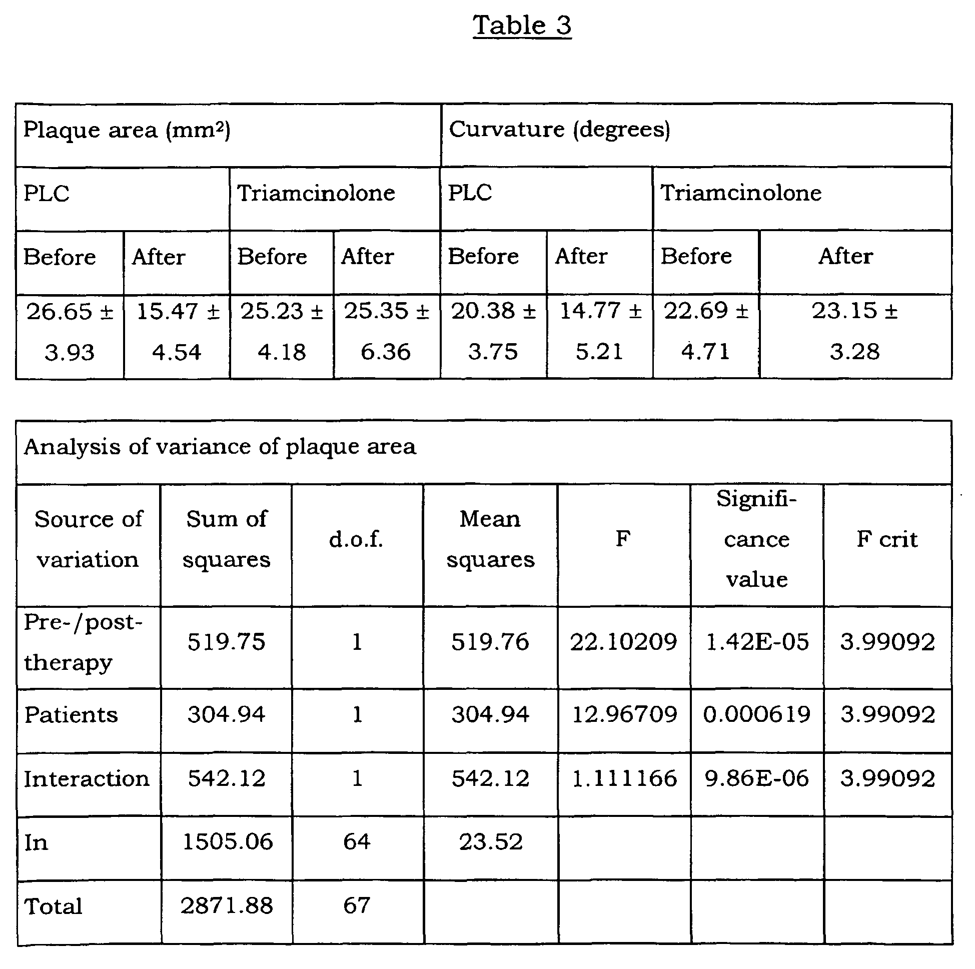

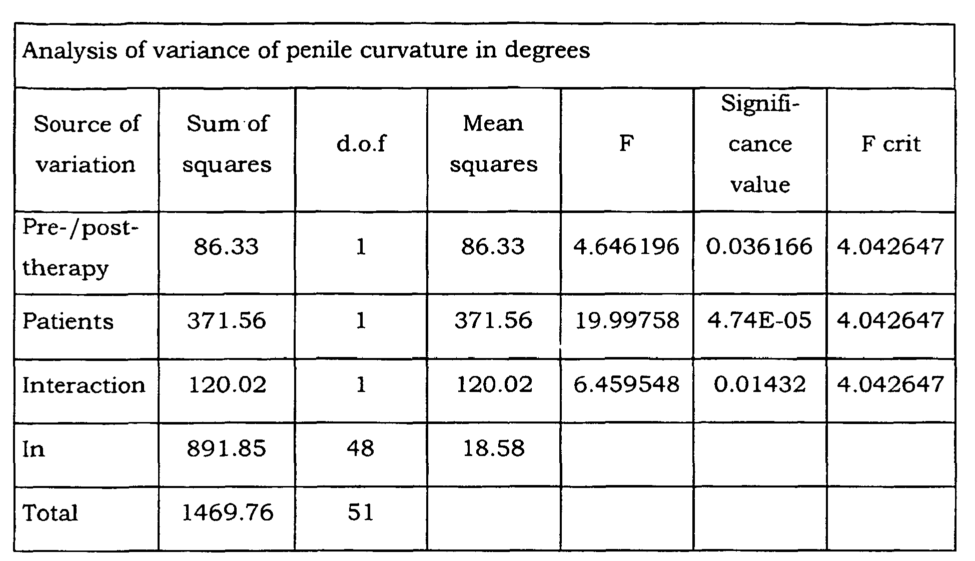

The examples continue with the use of oral propionyl L-carnitine combined with verapamil. These studies were conducted in most of the patients tested and therefore the analysis could be carried out on a large number of variables.

### Example 4

### Advanced La Peyronie's disease

The study was conducted according to a randomised double-blind protocol.

In all, a total of 60 patients with advanced La Peyronie's disease were examined (mean age 59 years, range 42-64 years) and randomised to two groups of 30. The disease was diagnosed and studied by means of history taking, physical examination, pharmacologically induced erection, photography of the erect penis, basal and dynamic colour Doppler ultrasonography of the penile cavernous arteries, and administration of the "International Index of Erectile Function" (I.I.E.F. 15) Questionnaire (*Rosen R.C., et al.: Urology 49: 822-830, 1997*). The patients were submitted to 10 intraplaque infiltrations (1 infiltration a week) with 10 mg of verapamil (*Levine L.A.: J. Urol. 158: 1395-1399, 1997*). The first group received propionyl L-carnitine 1 x 2 g/day for 3 months, and the second group tamoxifen 20 x 2 mg/day for 3 months. The data were collected 6 months after the end of therapy when the initial history taking and semeiological examination were repeated.

### Example 5

### La Peyronie's disease resistant to other therapies

The study was conducted with a before-after prospective design. In this last group of subjects it proved impossible to adopt a case-control design owing to the large number of treatments to which they had been subjected.

In all, a total of 15 patients (mean age 56, range 39-63) were treated for La Peyronie's disease which continued to progress despite previous therapies. Details of the previous therapies are as follows: intraplaque verapamil (I.V.) administration according to the protocol proposed by Levine (*ibid.*; tamoxifen (T) 20 x 2 mg/day for 3 months; vitamin E (E) 200-400 mg/ day for 3 months; extracorporeal shock wave therapy (E.C.S.W.), delivered using the Minilith SLI device, with 3000-4000 shocks per session for 6 sessions; iontophoresis (Itf) administered in 12 sessions (3 sessions a week) using verapamil and dexamethasone at the positive pole for 20 minutes. Five patients had been treated with IV+Itf+ECSW, 5 patients with (IV+T)+Itf+ECSW, 2 with IV+(IV+E)+Itf+ECSW, 1 with (IV+T)+(IV+E)+Itf+ECSW and 2 with (IV+E)+Itf+ECSW.

The disease was diagnosed and studied as indicated above. The patients were submitted to 10 intraplaque infiltrations (1 infiltration per week) with 10 mg of verapamil and to the oral administration of propionyl L-carnitine 1 x 2 g/day for 3 months. The data were collected 6 months after the end of the therapy when the initial history taking and semeiological examination were repeated.

The following variables were compared between groups and/or before and after therapy.
I. Pain on erection, quantified according to the international pain scale (*Beers M.H., Fietcher M.B.: The Merck Manual. 17th edition*. *West Point: Merck and Co. 1999*) (0 = no pain; 1 = mild pain; 2 = moderate pain; 3 = severe pain) with the results classified as positive or negative according to whether the score decreases or increases;
II. Penile curvature measured in degrees on a photograph taken in the outpatients' department during a pharmacologically induced full erection;
III. Plaque area measured in mm² by ultrasonography performed during a pharmacologically induced full erection;
IV. I.I.E.F. 15 score;
V. Peak systolic velocity (PSV) (cm/sec), end-diastolic velocity (EDV) (cm/sec) and the resistivity index (RI) of the right and left cavernous arteries using dynamic Doppler ultrasonography;
VI. Progression of the disease defined as an increase in the curvature and/or plaques and/or EDV and/or a reduction of the RI and/or PSV and/or I.I.E.F. 15 score, the results being defined as absence or presence of disease progression;
VII. Need for penis straightening surgery and/or for straightening and a prosthesis, the results being classified as presence or absence of any such need;
VIII. Side effects classified as presence or absence of side effects.

### Data analysis

The data relating to pain, disease progression, need for surgery and side effects were compared before and 6 months after therapy using the chi-square test. Differences in plaque area, penile curvature, I.I.E.F. 15 score, PSV, EDV and RI were calculated between groups and 6 months after therapy with 2 x 2 factorial analysis of variance in the case of advanced La Peyronie's disease, or with analysis of variance for randomised blocks (1 patient = 1 block) in patients with La Peyronie's disease resistant to therapy in order to check for individual variables that might have altered the analysis. The RI data were subjected to angular transformation [sin⁻¹ (p/100)], whereas in all other cases natural data were used (*Armitage P.: Statistical Methods in Medical Research. London: Blackwell Scientific Publications 1971*)*.*

### Advanced La Peyronie's disease

29 (96.7%) patients in group 1 presented a reduction of pain and only one (3.3%) showed no such reduction; exactly the same results were obtained in group 2 (chi-square = 0, P not significant).

The combination of verapamil plus propionyl L-carnitine was found to significantly reduce penile curvature (Table 4) and plaque area (Table 5) and to significantly increase the I.I.E.F. 15 score (Table 6). The effects of verapamil plus propionyl L-carnitine on the right and left cavernous arteries are presented in Tables 7 and 8, respectively. This pharmacological combination proved capable of significantly reducing EDV and increasing the RI. The combination of tamoxifen and verapamil had no significant effect on any of these variables. Neither of the two combinations showed any activity on PSV. One patient out of 30 in group 1 required surgery, while in group 2 an operation was recommended in 8 out of 30 cases (chi-square = 4.615; P<0.05). None of the patients in group 1 presented progression of the disease, whereas there were 6 cases of disease progression in group 2 (chi-square = 4.615; P<0.05). No side effects were observed in group 1 as against 6 cases in group 2 (chi-square = 4.615; P<0.05), namely, 3 cases of slight epigastric pain and 3 of slight loss of libido. None of the patients had to discontinue the therapy owing to side effects.

### La Peyronie's disease resistant to therapies

Nine patients out of 15 were still experiencing pain before being administered intraplaque verapamil and oral propionyl L-carnitine; after this therapy only 1 out of 15 had pain (chi-square = 7.35, *P*<0.01). The combination of verapamil and propionyl L-carnitine was found to significantly reduce penile curvature (Table 9) and plaque area (Table 10), and to significantly increase the I.I.E.F. 15 score (Table 11). The effects of verapamil and propionyl L-carnitine on the right and left cavernous arteries are presented in Tables 12 and 13, respectively. This pharmacological combination proved capable of significantly reducing EDV and increasing the RI, whereas it had no effect on PSV. Three patients required surgery and 1 presented progression of the disease.

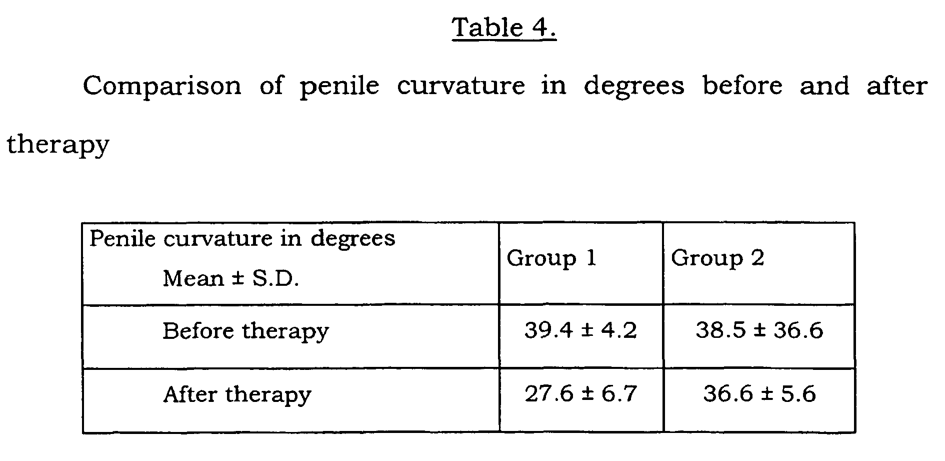

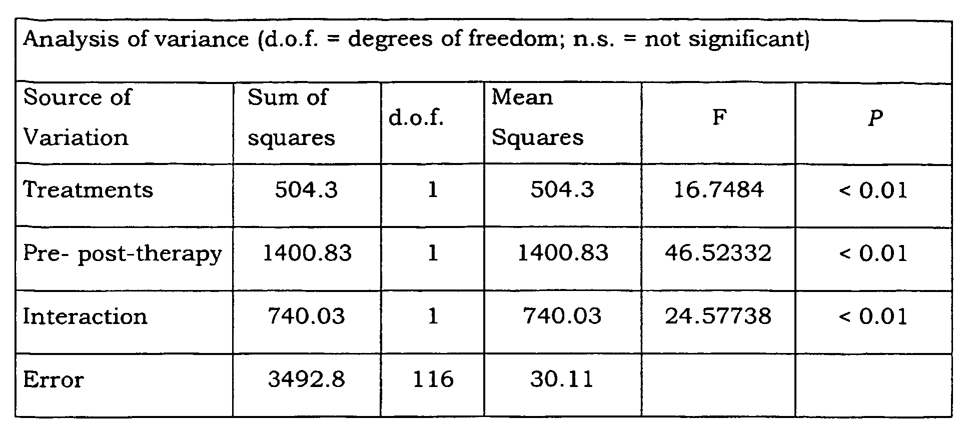

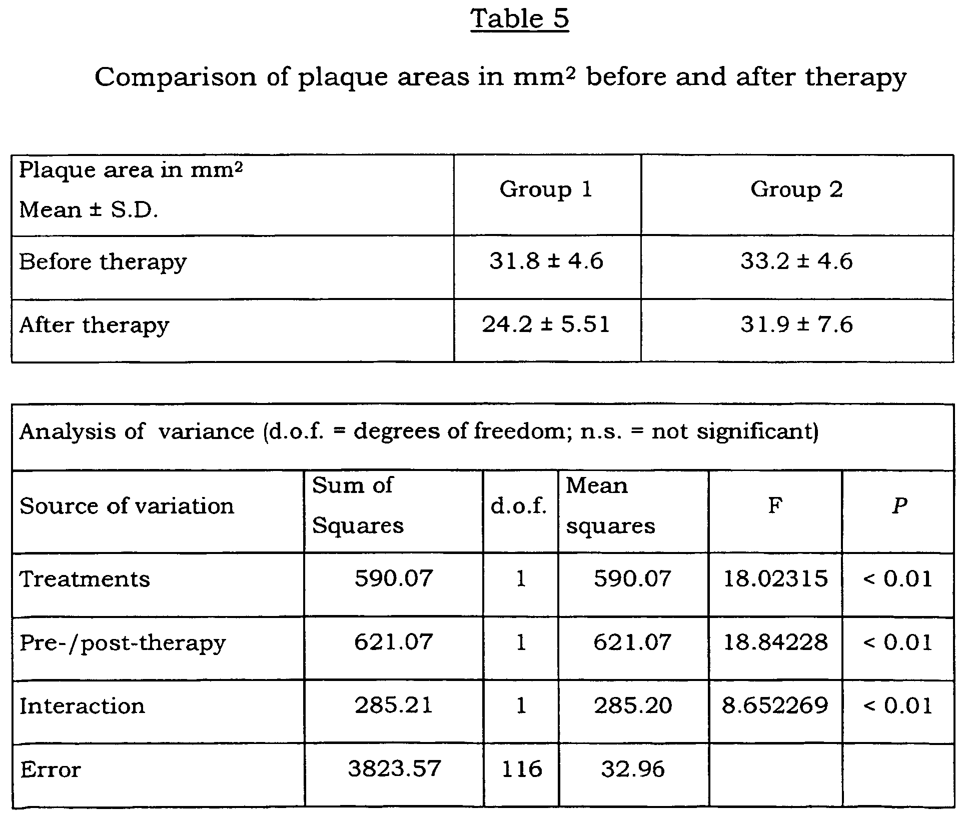

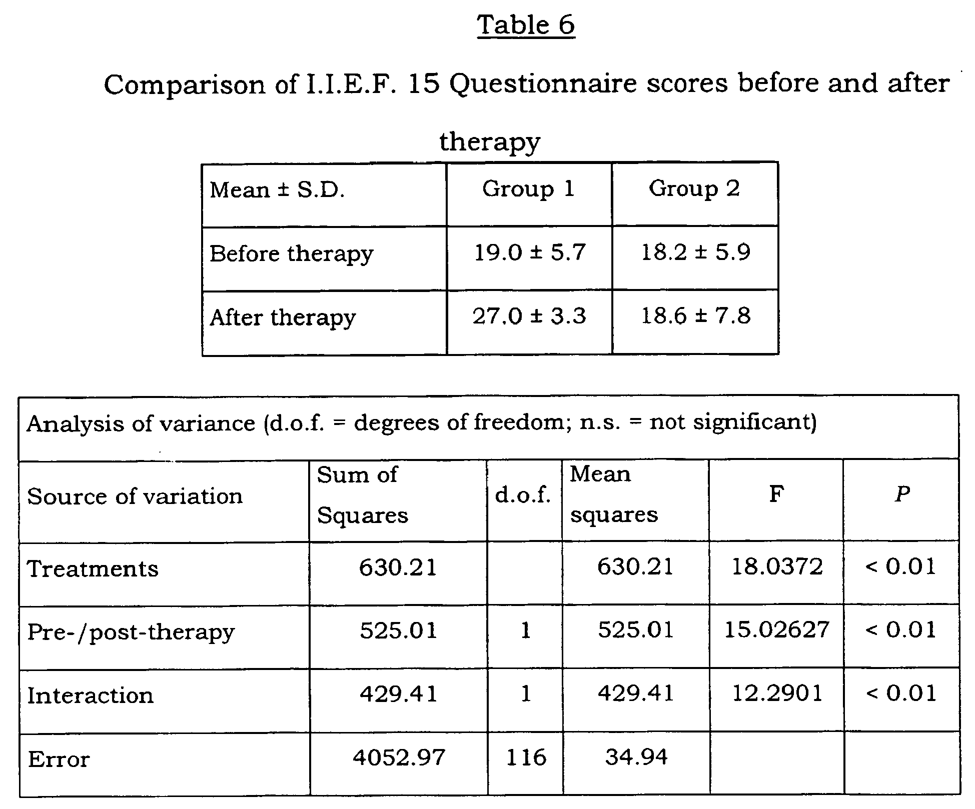

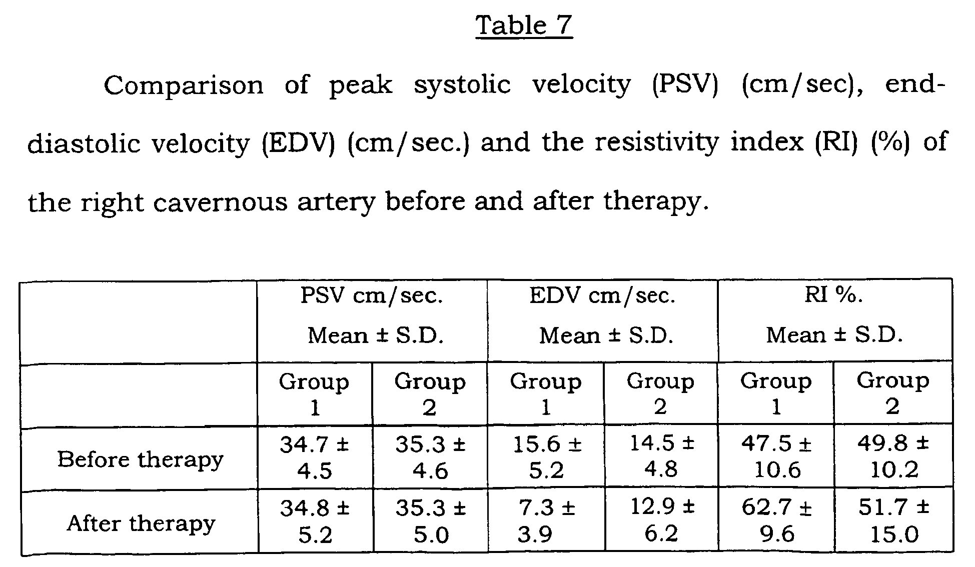

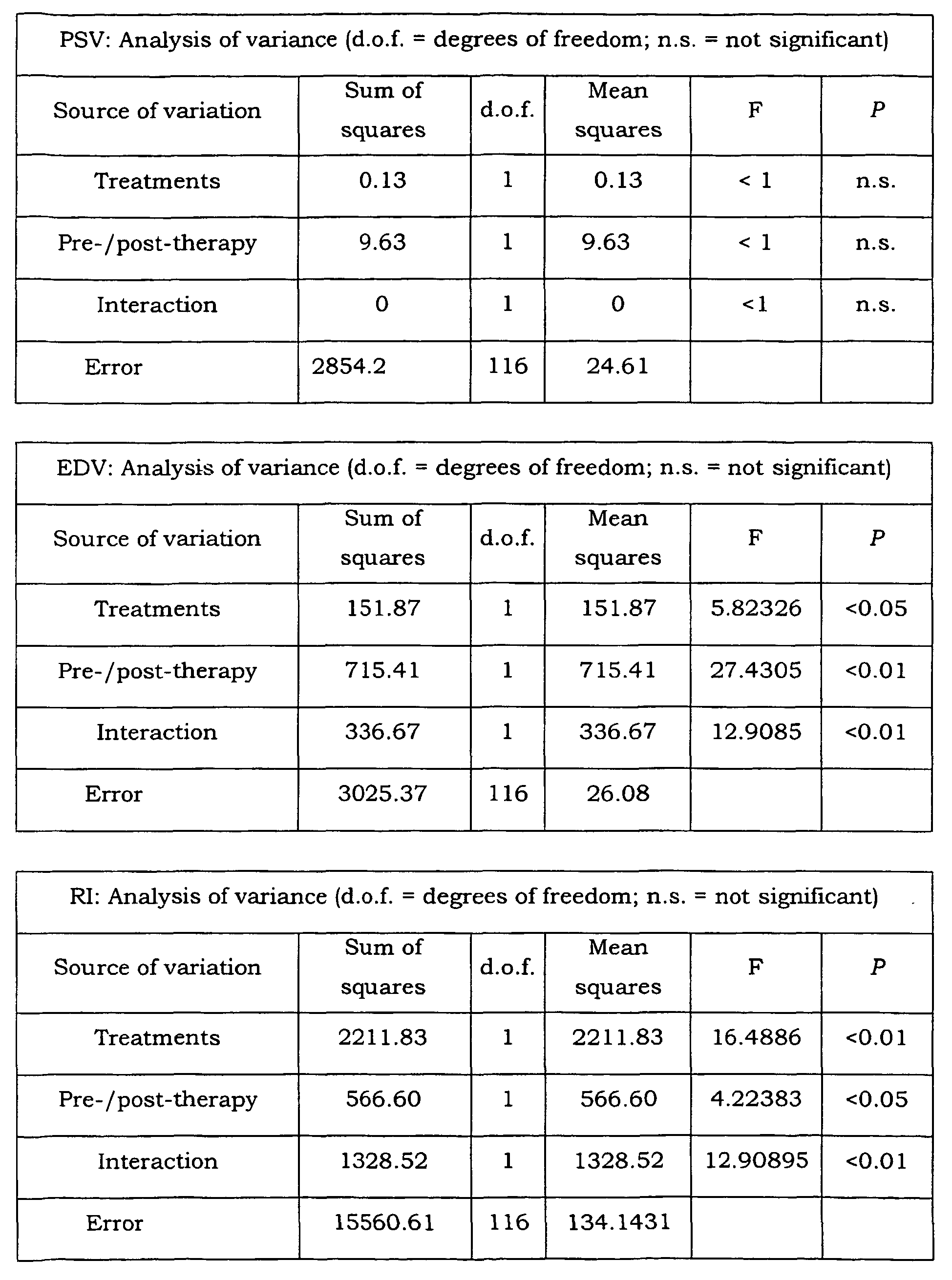

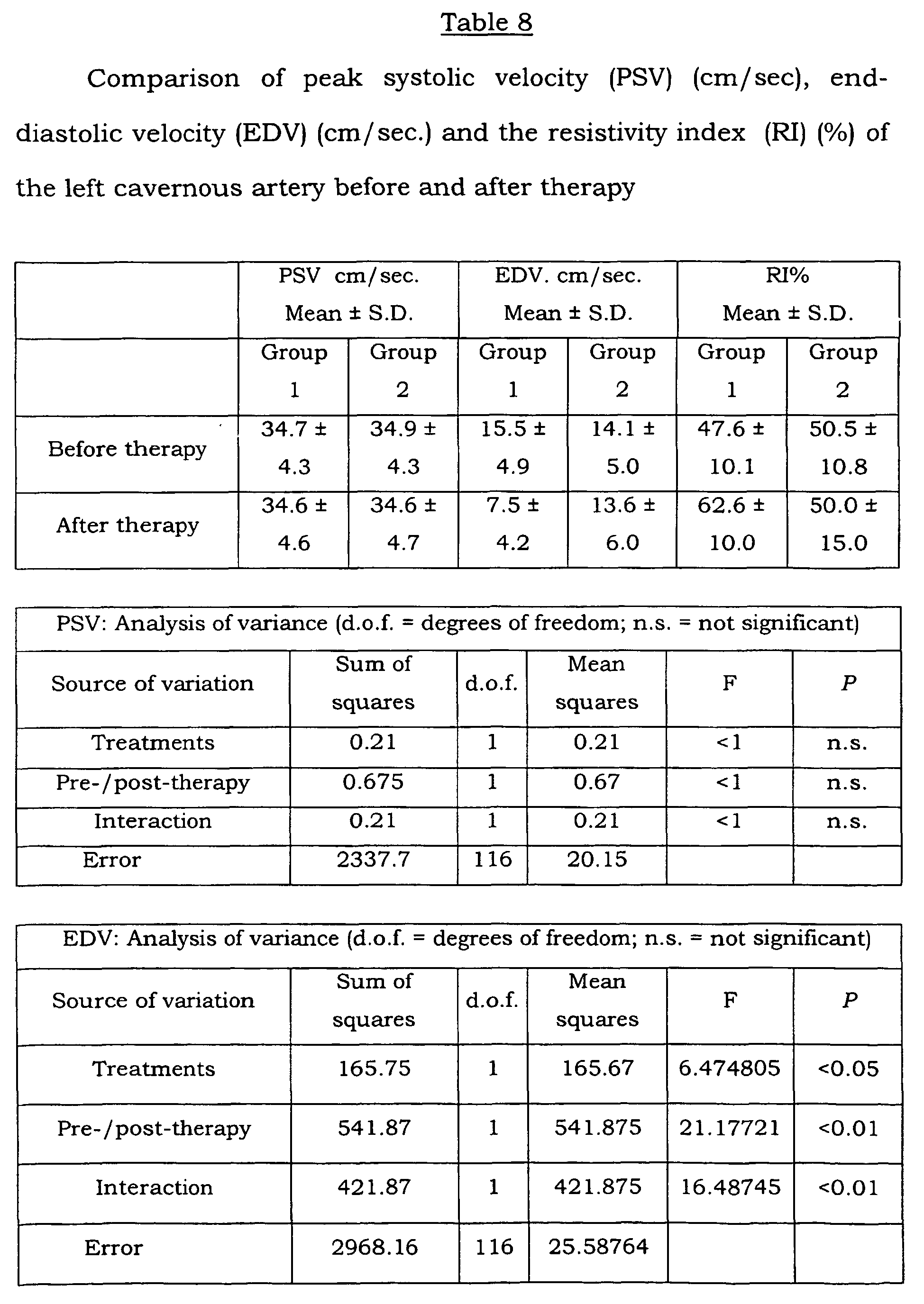

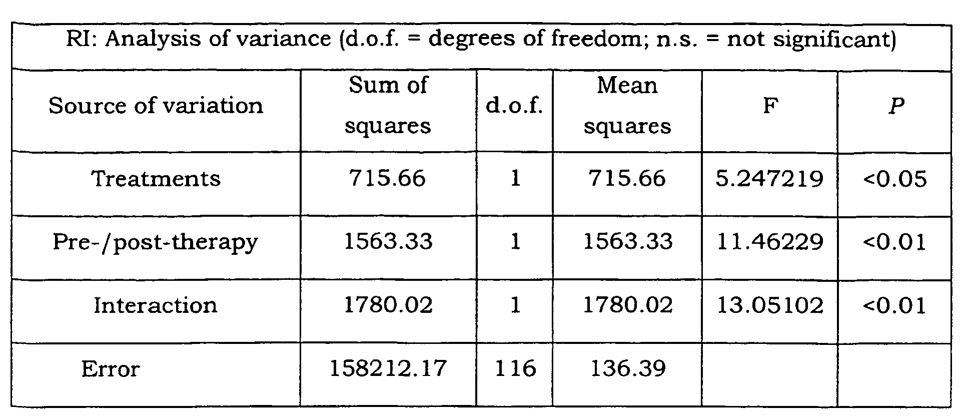

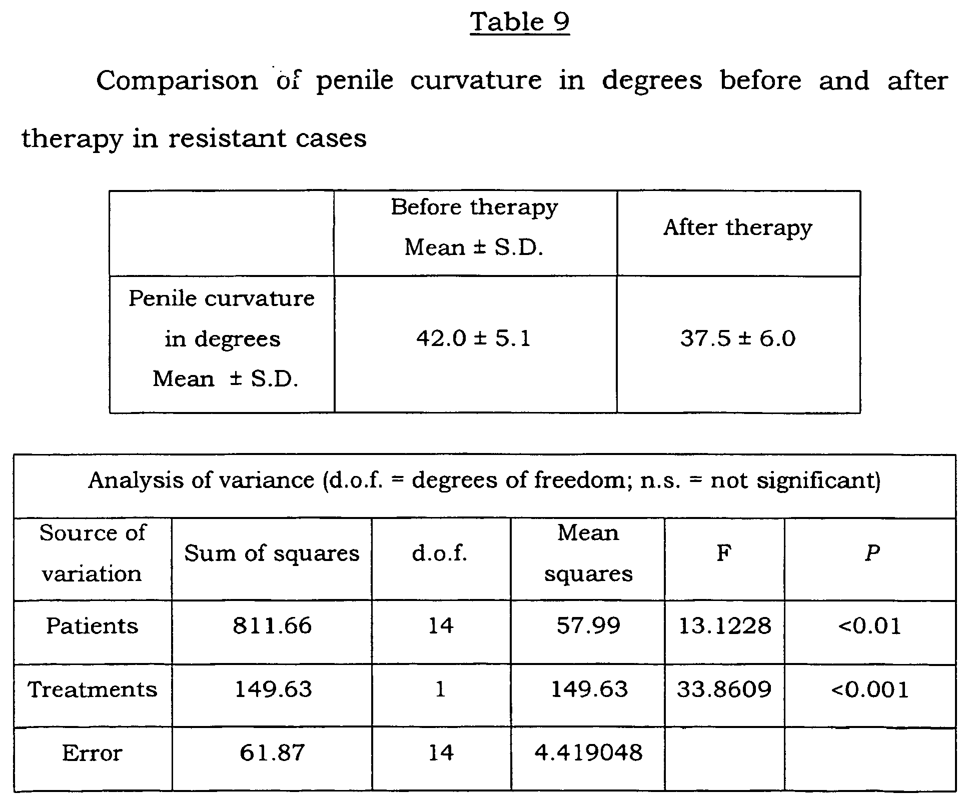

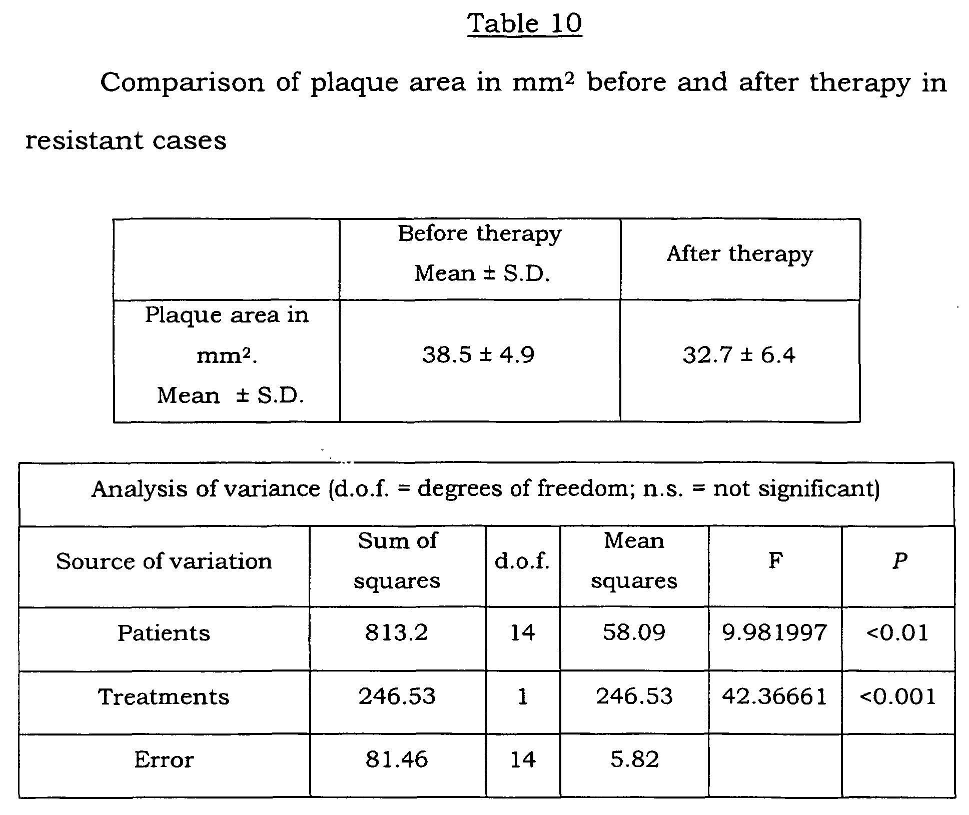

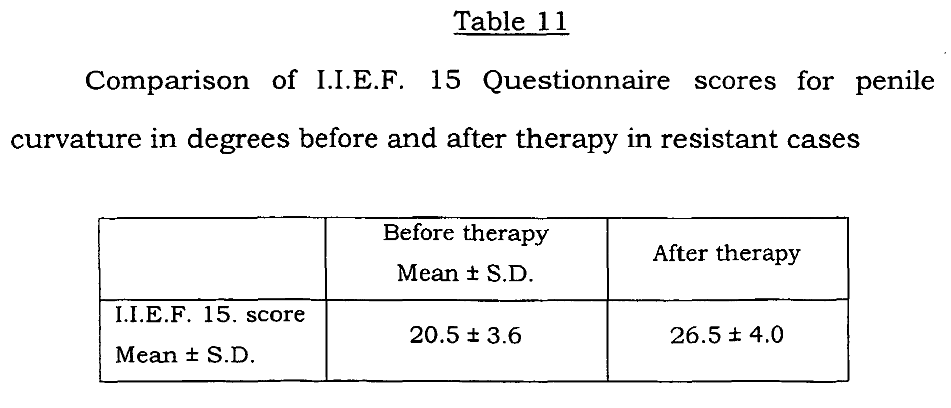

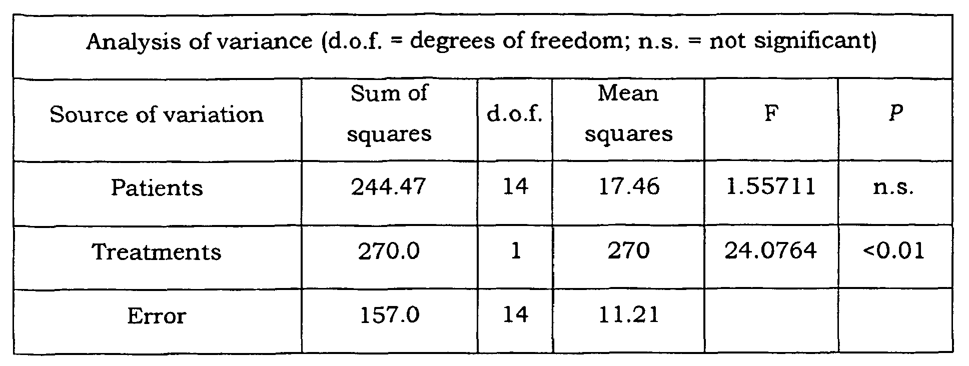

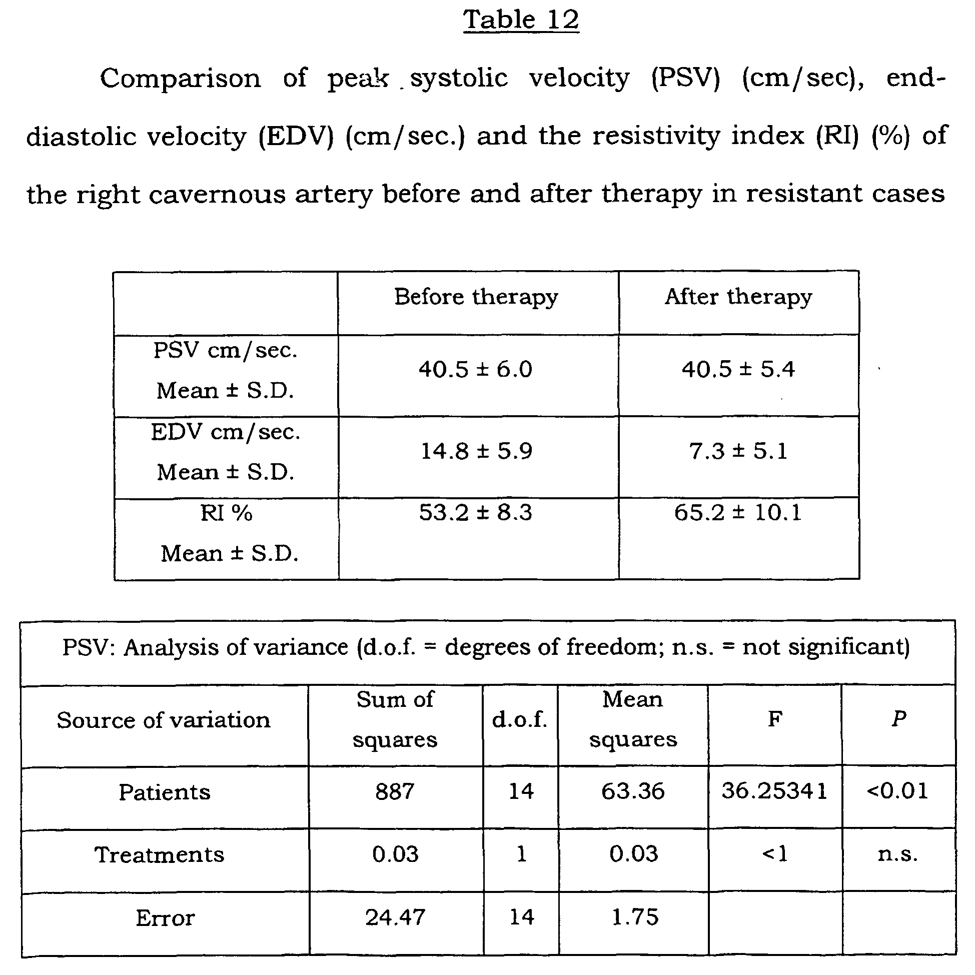

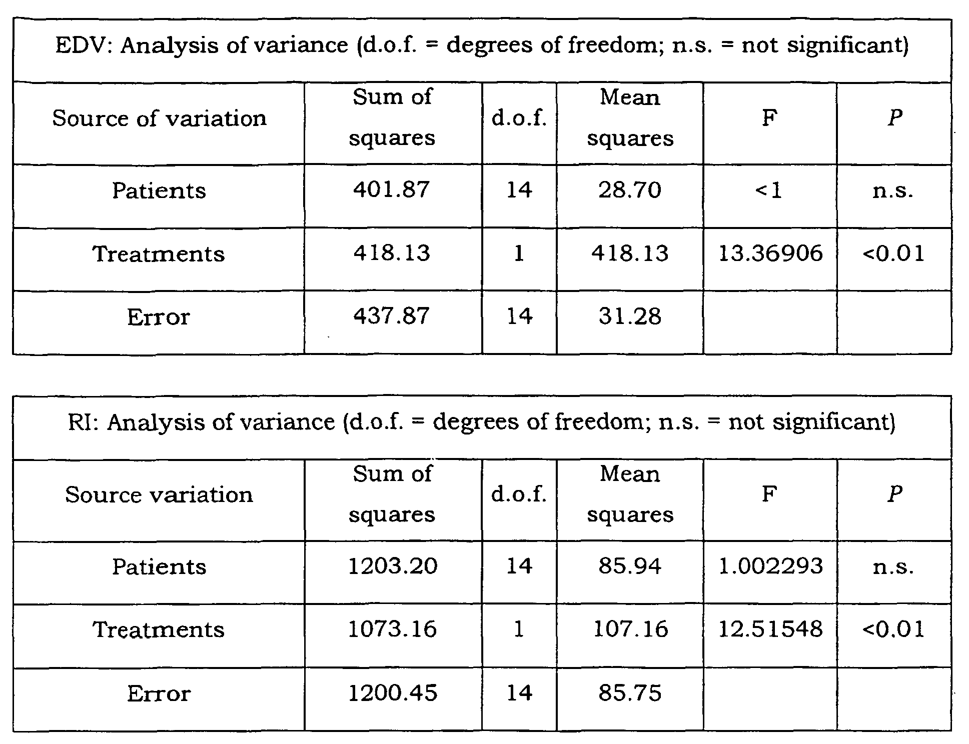

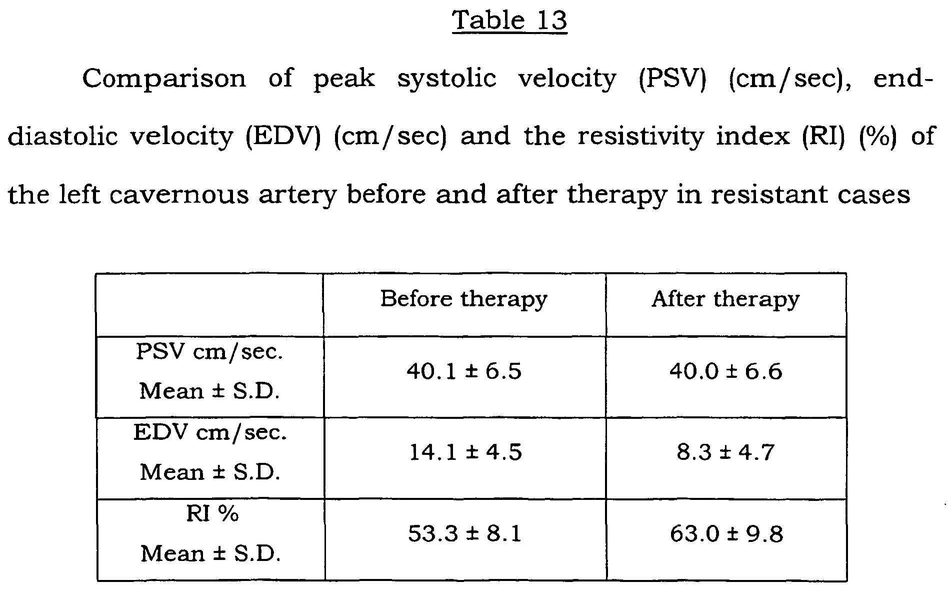

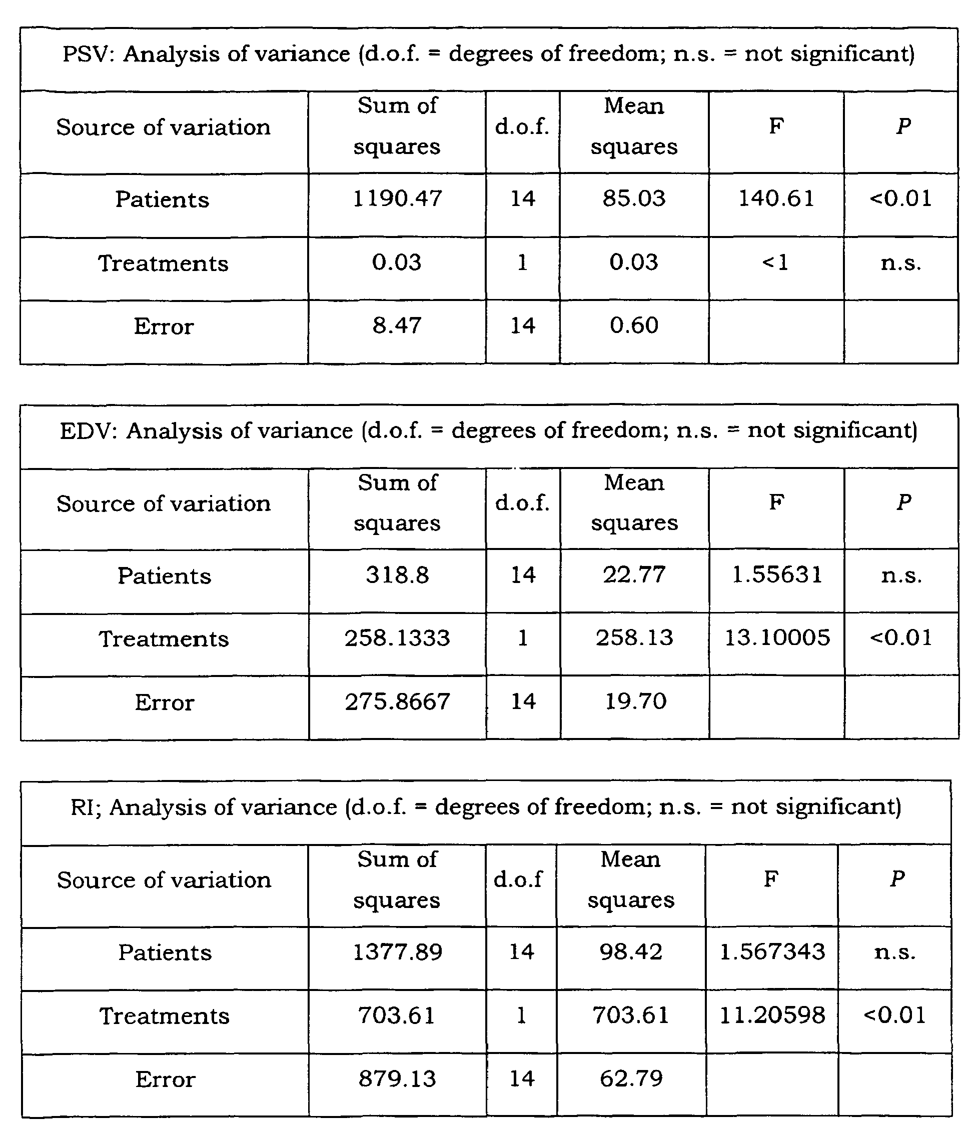

Propionyl L-carnitine proved significantly more active than combined tamoxifen and verapamil in relation to almost all the data characterising the disease and presented significantly fewer side effects. The apparent lack of activity of tamoxifen plus verapamil is due to the fact that the analysis of variance considers the entire panel of results embracing patients that improve, patients that remain stable and patients in whom the disease progresses. In actual fact, the combination of tamoxifen and verapamil arrested the progression of advanced La Peyronie's disease in 80% of cases (propionyl L-carnitine plus verapamil achieved significantly better results); advanced La Peyronie's disease, if left untreated, will progress in 90% of cases.

Advanced La Peyronie's disease is often associated with an erectile deficit (low I.I.E.F. 15 score). It is of no importance that neither of the pharmacological combinations proved effective on PSV, which is an indicator of patency of the arteries, since these are generally unimpaired in La Peyronie's disease. What is important, on the other hand, is the major activity on EDV, which measures venous resistance: the lower the EDV, the higher the resistance of the veins to blood flow or, in other words, the higher the resistivity index which is calculated as follows: R.I. = PSV-EDV/PSV x 100. The fact that the erectile activity of propionyl L-carnitine and verapamil manifested itself in the form of an action on EDV confirms the assumption that the erectile deficit of Petronie's disease is secondary to a venous deficiency.

In parallel tests the combination of propionyl L-carnitine plus verapamil proved significantly more active than the combination of acetyl L-carnitine plus verapamil, as demonstrated by the next example.

### Example 6

20 subjects (mean age 48, range 40-61) with induratio penis plastica at an advanced chronic stage were randomised to two groups of 10 patients each. The first group was submitted to therapy with oral propionyl L-carnitine (PLC) 1 x 2 g/day 3 months and intraplaque verapamil 10 mg (10 infiltrations, 1 a week), while the second group was submitted to therapy with oral acetyl L-carnitine (ALC) 1 x 2 g/day for 3 months + intraplaque verapamil 10 mg (10 infiltrations, 1 a week). The variables measured were plaque area by dynamic colour Doppler penile ultrasonography. In view of the limited number of subjects in the sample it was not possible to carry out any analysis of other data; in any event, the size of the plaque is the most important variable when monitoring La Peyronie's disease, since all the other symptoms depend on the extent of the plaque (*Belgrano, ibid.*). It was found that propionyl L-carnitine plus intraplaque verapamil reduce the plaque area to a significantly greater extent than acetyl L-carnitine plus intraplaque verapamil.

The results are presented in Table 14, where the data are expressed as mean ± standard deviation (S.D.).

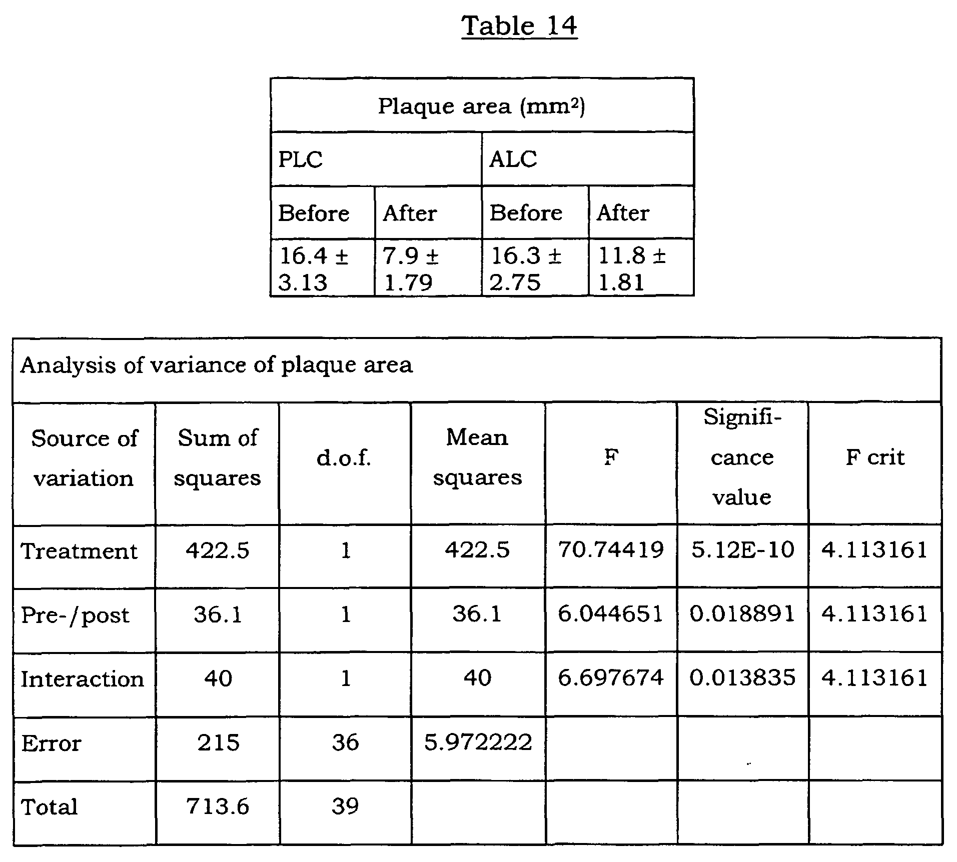

Propionyl L-carnitine, particularly in combination with verapamil, is the only drug to have proved efficacious in the therapy of resistant La Peyronie's disease which progresses despite previous therapies.

Propionyl L-carnitine is the only orally administered drug to have proved capable of boosting the action of intraplaque verapamil in the therapy of advanced La Peyronie's disease.

## Claims

1. Use of propionyl L-carnitine or one of its pharmaceutically acceptable salts for the preparation of a medicine useful for the treatment of La Peyronie's disease.

2. Use according to claim 1, in which said medicine is suitable for oral administration.

3. Use according to claim 1, in which said medicine is suitable for intramuscular, intravenous and intraplaque administration.

4. Use according to claim 1, 2 or 3, in which said La Peyronie's disease is at an advanced stage.

5. Use according to claim 1, 2 o 3, in which said La Peyronie's disease is resistant to conventional therapies.

6. Combination of propionyl L-carnitine, or one of its pharmaceutically acceptable salts, and an active ingredient useful for the treatment of La Peyronie's disease selected from the group consisting of tamoxifen, triamcinolone and verapamil.

7. Pharmaceutical composition containing the combination of claim 6 and vehicles and or excipients, if any.

8. Pharmaceutical composition of claim 7, in which said combination is suitable for the co-ordinated use.

9. Use of an active ingredient useful for the treatment of La Peyronie's disease, in combination with propionyl L-carnitine or one of its pharmaceutically acceptable salts, for the preparation of a medicine useful for the treatment of La Peyronie's disease.

10. Use according to claim 9, where said active ingredient is selected from the group consisting of tocopherols, vitamin E, allopurinol, potassium amino-benzoate, tamoxifen, immunomodulators, triamcinolone, and verapamil.

11. Use according to claim 9, in which said medicine is suitable for oral, intramuscular, intravenous and intraplaque administration.

12. Use according to claim 9, in which said medicine is suitable for the co-ordinated use of said combination.

13. Use according to claim 9, in which said medicine is suitable for the oral administration of propionyl L-carnitine and for the intraplaque administration of verapamil.

14. Use according to claim 9, in which said medicine is suitable for the oral administration of 1 g of propionyl L-carnitine, or an equivalent amount of one of its pharmaceutically acceptable salts, per dosage unit, and for the intraplaque administration of 10 mg of verapamil.

15. Use according to claim 9, in which said La Peyronie's disease is at an advanced stage.

16. Use according to claim 9, in which said La Peyronie's disease is resistant to conventional therapies.

## Patentansprüche

1. Verwendung von Propionyl-L-carnitin oder eines seiner pharmazeutisch akzeptablen Salze für die Herstellung einer Medizin, die für die Behandlung der Penisinduration geeignet ist.

2. Verwendung gemäß Anspruch 1, wobei die Medizin für eine orale Verabreichung geeignet ist.

3. Verwendung gemäß Anspruch 1, wobei die Medizin für eine intramuskuläre, intravenöse oder intraplaqueverabreichung geeignet ist.

4. Verwendung gemäß Anspruch 1, 2 oder 3, wobei sich die Penisinduration in einem fortgeschrittenen Zustand befindet.

5. Verwendung gemäß Anspruch 1, 2 oder 3, wobei die Penisinduration gegen konventionellen Therapien resistent ist.

6. Kombination von Propionyl-L-carnitin oder eines seiner pharmazeutisch akzeptablen Salze und einem Wirkstoff, der für die Behandlung der Penisinduration nützlich ist, ausgewählt aus der Gruppe bestehend aus Tamoxiferi, Triamcinolon und Verapamil.

7. Pharmazeutische Zusammensetzung enthaltend die Kombination gemäß Anspruch 6 und Vehikel und/oder Exzipienten, falls solche vorhanden.

8. Pharmazeutische Zusammensetzung gemäß Anspruch 7, wobei die Kombination für die koordinierte Verwendung geeignet ist.

9. Verwendung eines Wirkstoffs, der für die Behandlung der Penisinduration nützlich ist, in Kombination mit Propionyl-L-carnitin oder eines seiner pharmazeutisch akzeptablen Salze für die Herstellung einer Medizin, die für die Behandlung der Penisinduration nützlich ist.

10. Verwendung gemäß Anspruch 9, wobei der Wirkstoff ausgewählt ist aus der Gruppe bestehend aus Tocopherolen, Vitamin E, Allopurinol, Kaliumaminobenzoat, Tamoxifen, Immunmodulatoren, Triamcinolon und Verapamil.

11. Verwendung gemäß Anspruch 9, wobei die Medizin für die orale, intramuskuläre, intravenöse und Intraplaqueverabreichung geeignet ist.

12. Verwendung gemäß Anspruch 9, wobei die Medizin für die koordinierte Verwendung der Kombination geeignet ist.

13. Verwendung gemäß Anspruch 9, wobei die Medizin für die orale Verabreichung von Propionyl-L-carnitin und für die Intraplaqueverabreichung von Verapamil geeignet ist.

14. Verwendung gemäß Anspruch 9, wobei die Medizin für die orale Verabreichung von 1 g Propionyl-L-carnitin oder einer äquivalenten Menge von einem der pharmazeutisch akzeptablen Salze davon pro Dosierungseinheit und für die Intraplaqueverabreichung von 10 mg Verapamil geeignet ist.

15. Verwendung gemäß Anspruch 9, wobei sich die Penisinduration in einem fortgeschrittenen Stadium befindet.

16. Verwendung gemäß Anspruch 9, wobei die Penisinduration gegenüber konventionellen Therapien resistent ist.

## Revendications

1. Utilisation de propionyl-L-carnitine ou de l'un de ses sels pharmaceutiquement acceptables pour la préparation d'un médicament destiné au traitement de la maladie de La Peyronie.

2. Utilisation selon la revendication 1, dans laquelle ledit médicament est adapté à une administration orale.

3. Utilisation selon la revendication 1, dans laquelle ledit médicament est adapté à une administration intramusculaire, intraveineuse et intra-plaques.

4. Utilisation selon la revendication 1, 2 ou 3, dans laquelle ladite maladie de La Peyronie est à un stade avancé.

5. Utilisation selon la revendication 1, 2 ou 3, dans laquelle ladite maladie de La Peyronie est résistante aux thérapies conventionnelles.

6. Combinaison de propionyl-L-carnitine, ou de l'un de ses sels pharmaceutiquement acceptables, et d'un ingrédient actif, utile pour le traitement de la maladie de La Peyronie, choisi parmi le groupe constitué du tamoxifene, de la triamcinolone et du vérapamil.

7. Composition pharmaceutique contenant la combinaison de la revendication 6 et éventuellement des véhicules et/ou des excipients.

8. Composition pharmaceutique selon la revendication 7, dans laquelle ladite combinaison est adaptée à une utilisation coordonnée.

9. Utilisation d'un ingrédient actif utile pour le traitement de la maladie de La Peyronie, en combinaison avec de la propionyl-L-carnitine, ou l'un de ses sels pharmaceutiquement acceptables, pour la préparation d'un médicament destiné au traitement de la maladie de La Peyronie.

10. Utilisation selon la revendication 9, dans laquelle ledit ingrédient actif est choisi dans le groupe constitué des tocophérols, de la vitamine E, de ratiopurinol, de l'amino-benzoate de potassium, du tamoxifene, d'unmunomodulateurs, de la triamcinolone et du vérapamil.

11. Utilisation selon la revendication 9, dans laquelle ledit médicament est adapté à une administration orale, intramusculaire, intraveineuse et intra-plaques.

12. Utilisation selon la revendication 9, dans laquelle ledit médicament est adapté à une utilisation coordonnée de ladite combinaison.

13. Utilisation selon la revendication 9, dans laquelle ledit médicament est adapté à l'administration orale de la propionyl-L-carnitine et à l'administration intra-plaques de vérapamil.

14. Utilisation selon la revendication 9, dans laquelle ledit médicament est adapté à l'administration orale de 1 g de propionyl-L-carnitine, ou d'une quantité équivalente de l'un de ses sels pharmaceutiquement acceptables, par dose unitaire, et à l'administration intra-plaques de 10 mg de vérapamil.

15. Utilisation selon la revendication 9, dans laquelle ladite maladie de La Peyronie est à un stade avancé.

16. Utilisation selon la revendication 9, dans laquelle ladite maladie de La Peyronie est résistante aux thérapies conventionnelles.
